# EUROPÄISCHE PATENTSCHRIFT

(11) **EP 1 560 520 B1**
(45) Veröffentlichungstag und Bekanntmachung des Hinweises auf die Patenterteilung: **30.11.2011**
(21) Anmeldenummer: 03767386.0
(22) Anmeldetag: 27.10.2003
(51) Int. Cl.: A61B 6/14, G03B 42/04

(54) **AUFBISSVORRICHTUNG ZUR VERWENDUNG MIT EINEM PANORAMA-RÖNTGENGERÄT**
BITE DEVICE USED WITH A PANORAMA X-RAY DEVICE
DISPOSITIF A MORDRE DESTINE A ETRE UTILISE AVEC UN APPAREIL DE RADIOGRAPHIE PANORAMIQUE

(30) Priorität: 25.10.2002 DE 10250005
(43) Veröffentlichungstag der Anmeldung: 10.08.2005
(73) Patentinhaber: Sirona Dental Systems GmbH, 64625 Bensheim (DE)
(72) Erfinder: STOECKL, Klaus, 64625 Bensheim (DE)
(74) Vertreter: Sommer, Peter
(86) Internationale Anmeldenummer: PCT/DE2003/003572
(87) Internationale Veröffentlichungsnummer: WO 2004/039261

(56) Entgegenhaltungen:
- WO-A-02/086619
- US-A- 4 176 278
- US-A- 6 118 842
- US-B1- 6 190 042
- US-B1- 6 424 694

## Beschreibung

### Technisches Gebiet

Die Erfindung betrifft eine Vorrichtung zur korrekten Positionierung eines Patienten bei einer Aufnahme mit einem medizinischen Panorama-Röntgenaufnahmegerät.

### Stand der Technik

Beim Aufnehmen einer Panorama-Röntgenaufnahme verläuft die Tomographiezone idealerweise durch den Kieferkammbogen des Patienten. Um eine hohe Aufnahmequalität zu erreichen, ist es erforderlich, die beiden Elemente Tomographiezone und Kieferkammbogen in möglichst gute räumliche Übereinstimmung zu bringen. Dabei kann die Tomographiezone auf Seiten des Panorama-Röntgengeräts prinzipiell innerhalb gewisser Grenzen auf die individuelle Form und Lage des Kiefers eines Patienten angepasst werden. Voraussetzung ist allerdings, dass diese dem Bediener bekannt sind. Dabei kommt besonders der Kenntnis folgender Merkmale Bedeutung zu:
- die räumliche Orientierung der Occlusalebene (Kauebene) bezüglich eines Bezugspunkts des Panorama-Röntgengeräts; und
- die individuelle anatomische Ausprägung des Kiefers, nach Größe, Form, Anomalien und dergleichen.

Herkömmlich wird der Kopf des Patienten für eine Panorama-Röntgenaufnahme zum Gerät über eine Patientenkopfpositionierung mit Stirnstütze und/oder Ohrolivenhalten, Zahnaufbiss, Nasion oder einer Kinnauflage fixiert. Die notwendige Ausrichtung des Kopfes erfolgt typischerweise über Lichtlinien, die auf den Kopf des Patienten projiziert werden und die die Frankfurter Horizontale und die Medianebenen abbilden. Die Frankfurter Horizontale verläuft durch das rechte und linke Porion, den höchsten Punkt des Meatus acusticus externus, und das Orbitale, das den tiefsten Punkt des knöchernen Rands der Orbita darstellt. Die projizierte Medianebene verläuft als sagittale Ebene vom Scheitelpunkt über die Nasenmitte zum Kinn. Sie unterstützt die symmetrische Positionierung des Kopfes.

Die Lage der Kauebene wird nicht direkt erfasst, sondern nur indirekt über die Frankfurter Horizontale. Dazu wird über die vom Panorama-Röntgengerät auf den Kopf des Patienten projizierte Frankfurter Horizontale und die Schneidezähne im Aufbiss auf die Lage der Kauebene geschlossen. Die Korrelation zwischen Kauebene und Frankfurter Horizontale ist seitens der Anatomie zwar allgemein anerkannt, Positionierungsfehler bezüglich der Frankfurter Horizontale des Patienten sind dabei jedoch nicht ausgeschlossen.

Um die individuelle Größe des Kiefers des Patienten zu bestimmen, kann optional eine Stirnweitenmessung durchgeführt werden. Die Ableitung der Kiefergröße ist jedoch nicht in hinreichendem Maß zuverlässig.

Auch existiert bislang kein verlässliches Verfahren zur Bestimmung der Form des Kieferkammbogens und möglicher Anomalien des Kiefers.

Heutige Panorama-Röntgengeräte bieten als Folge nur wenige Modifikationsmöglichkeiten, da der Bediener solche Einstellungsmöglichkeiten aufgrund der unzureichenden Kenntnis der individuellen Ausprägung des Kiefers eines Patienten ohnehin nicht nutzen könnte.

Aus der US 6,424,694 B1 ist ein Positionierapparat bekannt, der zur Erzeugung transversaler Schichtaufnahmen verwendet wird. Dieser Positionierapparat umfasst einen bezüglich dem Röntgengerät fest angeordneten Halter sowie eine verschwenkbare Platte, wobei Mittel vorgesehen sind, mit denen der Drehwinkel zwischen der Platte und dem Halter erfasst werden kann. Mit diesem Positionierapparat ist es möglich, die Neigung der Kauebene mittels der Verstellvorrichtung einzustellen. Allerdings ist der in D1 offenbarte Positionierapparat nur zur Erstellung transversaler Schichtaufnahmen geeignet.

Aus der US 6,118,842 ist Röntgeneinrichtung zur Erstellung von tomographischen Aufnahmen und von Panoramaschichtaufnahmen bekannt. Der Patientenkopf wird mittels einer horizontal und vertikal linear verfahrbaren Kinnstütze positioniert, wobei Eingabemittel vorgesehen sind, mit deren Hilfe gewünschte Positionen der Kinnstütze eingestellt werden können.

Die Position der Kinnstütze kann über Mittel zur Speicherung der Objektposition ausgelesen werden, wobei eine Änderung der Position über gesteuerte Motoren vorzunehmen ist, beispielsweise über Schrittmotoren.

Der Erfindung, wie sie in den Ansprüchen gekennzeichnet ist, liegt die Aufgabe zugrunde, eine Vorrichtung zum korrekten Positionieren eines Patienten bei einer Aufnahme mit einem medizinischen Panorama-Röntgenaufnahmegerät anzugeben, die eine einfache und robuste Erfassung und/oder Einstellung der Neigung der Kauebene eines Patienten erlaubt.

### Darstellung der Erfindung

Diese Aufgabe wird erfindungsgemäß durch die Aufbissvorrichtung nach Anspruch 1 gelöst. Vorteilhafte Ausgestaltungen der Erfindung sind Gegenstand der Unteransprüche.

Die Erfindung baut auf dem Stand der Technik dadurch auf, dass eine Aufbissvorrichtung ein zum Röntgengerät richtungsfest anordenbares Halterteil und eine gegen das Halterteil verschwenkbaren Platte enthält, die ein Aufbissteil aufweist, in das der Patient beißt. Die Aufbissvorrichtung enthält weiter Mittel zum Erfassen des mit der Neigung der Kauebene in Zusammenhang stehenden Schwenkwinkels α zwischen der Platte und dem Halterteil, wobei die Mittel zum Erfassen des Schwenkwinkels α in einem bei einer Röntgenaufnahme strahlenfreien Bereich der Aufbissvorrichtung angeordnet sind. Der von Röntgenstrahlen erfasste Bereich kann somit weitestgehend metallfrei gehalten werden.

Die Erfindung beruht somit auf dem Gedanken, die relative Lage der Kauebene des Patienten zum Gerät über die Lagebestimmung einer in der Kauebene liegenden Platte zu erfassen. Dazu wird der Winkel zwischen der Platte und einem Halterteil gemessen, das relativ zum Röntgengerät mit einer festen Ausrichtung anordenbar ist. Aus diesem Winkel kann dann ein Signal abgeleitet werden, das die Neigung der Kauebene angibt.

Die Mittel zum Erfassen des Schwenkwinkels α umfassen dabei zweckmäßig einen oder mehrere im Halterteil angeordnete Sensoren. Beispielsweise können die Sensoren durch eine im Halterteil angeordnete Lichtschranke gebildet sein, die die Position einer durch die Verschwenkung der Platte auf- und abbewegten Ausnehmung erfasst.

Die erfindungsgemäße Aufbissvorrichtung kann zweckmäßig Mittel zur Anzeige der Winkelstellung der verschwenkbaren Platte enthalten.

Weiterhin sind Antriebsmittel zur Höhenverstellung des Halteteils und damit zum Verschwenken der Platte in eine vorbestimmte Winkelposition vorgesehen. Dadurch ist eine Anpassung an die Körpergröße des Patienten möglich und der Patient kann gezwungen werden, die Neigung seines Kopfes der Neigung der Platte anzupassen, bei der eine gewünschte Winkelposition der Kauebene des Patienten relativ zum Röntgengerät erreicht ist.

Mit Vorteil fahren die Mittel zum Verschwenken der Platte die vorbestimmte Winkelposition automatisch an und halten bei Erreichen der vorbestimmten Winkelposition an.

Zusätzlich kann vorgesehen sein, dass die Mittel das Erreichen der vorbestimmten Winkelposition der Platte durch optische und/oder akustische Signale anzeigen.

In einer bevorzugten Ausgestaltung der erfindungsgemäßen Aufbissvorrichtung ist die schwenkbare Platte mit einer im Halterteil auf- und abbeweglichen Schiene verbunden, die eine Ausnehmung zur Positionsbestimmung der Schiene im Halterteil aufweist. Aus der Position der Ausnehmung im Halterteil kann dann auf den Schwenkwinkel α geschlossen werden.

Das Aufbissteil der Aufbissvorrichtung ist in einer zweckmäßigen Ausgestaltung aus hygienischen Gründen mit einer auswechselbaren Schutzhülle versehen.

Alternativ kann das Aufbissteil durch einen auswechselbaren Aufbisseinsatz gebildet sein. Der Aufbisseinsatz ist dabei bevorzugt aus einem weichen Material gebildet, insbesondere aus einem im Wesentlichen röntgenstrahlendurchlässigen Material. Als besonders gut geeignet hat sich die Fertigung des Aufbisseinsatzes aus geschlossenzelligem Ethylenschaum herausgestellt.

Der Aufbisseinsatz nimmt zweckmäßig einen Winkelbereich β des Kieferbogens auf, der zwischen 20° und 40°, insbesondere bei etwa 30° liegt. Dadurch wird ein seitliches Kippen oder eine Neigung des Kopfes des Patienten weitgehend vermieden.

In einer vorteilhaften Gestaltung weist der Aufbisseinsatz auf seiner Oberseite und seiner Unterseite eine Bissrille zur Aufnahme eines Teils des Zahnbogens von Ober- und Unterkiefer des Patienten auf.

Der Aufbisseinsatz ist mit Vorteil einstückig und entlang einer Faltkante klappbar ausgebildet. Zweckmäßig weist er an gegenüberliegenden Seiten einen sich keilförmig verjüngenden Vorsprung und eine entsprechende Vertiefung zur Aufnahme des Vorsprungs auf, zum lösbaren Anbringen des Aufbisseinsatzes an die schwenkbare Platte.

Weitere vorteilhafte Ausgestaltungen, Merkmale und Details der Erfindung ergeben sich aus den abhängigen Ansprüchen, der Beschreibung der Ausführungsbeispiele und den Zeichnungen.

### Kurzbeschreibung der Zeichnung

Die Erfindung soll nachfolgend anhand eines Ausführungsbeispiels im Zusammenhang mit den Zeichnungen näher erläutert werden. Es sind jeweils nur die für das Verständnis der Erfindung wesentlichen Elemente dargestellt. Dabei zeigt
- Fig. 1: eine schematische Darstellung einer PanoramaRöntgenaufnahmeeinrichtung;
- Fig. 2: in schematischer Darstellung die Ausrichtung der Tomographiezone der Röntgenaufnahmeeinrichtung auf den Kieferbogenkamm eines Patienten;
- Fig. 3: eine perspektivische Ansicht einer Aufbissvorrichtung nach einem Ausführungsbeispiel der Erfindung in schematischer Darstellung;
- Fig. 4: einen Schnitt durch die Aufbissvorrichtung von Fig. 3;
- Fig. 5: perspektivische Ansichten eines Aufbisseinsatzes für eine Aufbissvorrichtung nach einem Ausführungsbeispiel der Erfindung, gesehen aufgeklappt in (a) schräg von unten und in (b) schräg von oben;
- Fig. 6: einen Schnitt durch den Aufbisseinsatz von Fig. 5, gesehen entlang der Linie VI-VI; und
- Fig. 7: den Aufbisseinsatz von Fig. 5 in zusammengeklappter Stellung, wie er an der Aufbissvorrichtung der Fig. 3 angebracht ist.

### Ausführungsbeispiele

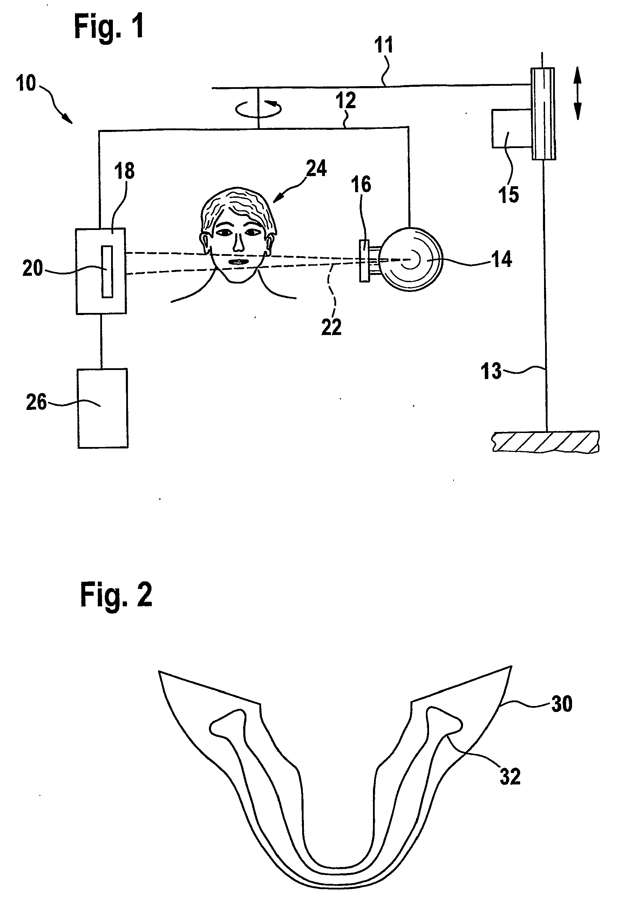

Fig. 1 zeigt in schematischer Darstellung eine Panorama-Röntgenaufnahmeeinrichtung 10, bei der eine schwenkbare Einheit 12 eine Strahlenquelle 14 mit einer Blende 16, und eine gegenüberliegende Detektorkamera 18 mit einer Blende 20 trägt. Der von der Strahlenquelle 14 ausgehende Röntgenstrahl 22 durchleuchtet den Kieferbereich des Kopfes 24 eines Patienten und erzeugt in der Detektorkamera 18 ein Bildsignal. Dieses wird einer Auswerteeinheit 26 zur Auswertung und Darstellung in an sich bekannter Weise zugeführt.

Die Dreheinheit 12 ist an einem Ausleger 11 dreh- bzw. schwenkbar befestigt, wobei der Ausleger 11 an einer Säule 13 höhenverstellbar gelagert ist. Zur Durchführung der Höhenverstellung ist ein Antrieb 15 vorgesehen. Auf diese Art und Weise können der Röntgenstrahler 14 und die Detektorkamera 18 auf die Größe des Patienten ausgerichtet werden.

Wesentlich für eine fehlerfreie Panorama-Röntgenaufnahme hoher Qualität ist die Ausrichtung der Tomographiezone 30 (Fig. 2) auf den Kieferbogenkamm 32 des Patienten. Fig. 2 zeigt eine perfekte räumliche Ausrichtung der beiden Elemente, die eine qualitativ einwandfreie Aufnahme sicherstellt. Eine unzureichende Ausrichtung kann dazu führen, dass eine Wiederholungsaufnahme notwendig wird, die zu einer erhöhten Strahlenbelastung des Patienten und zu zusätzlichem Material- und Zeitaufwand führt.

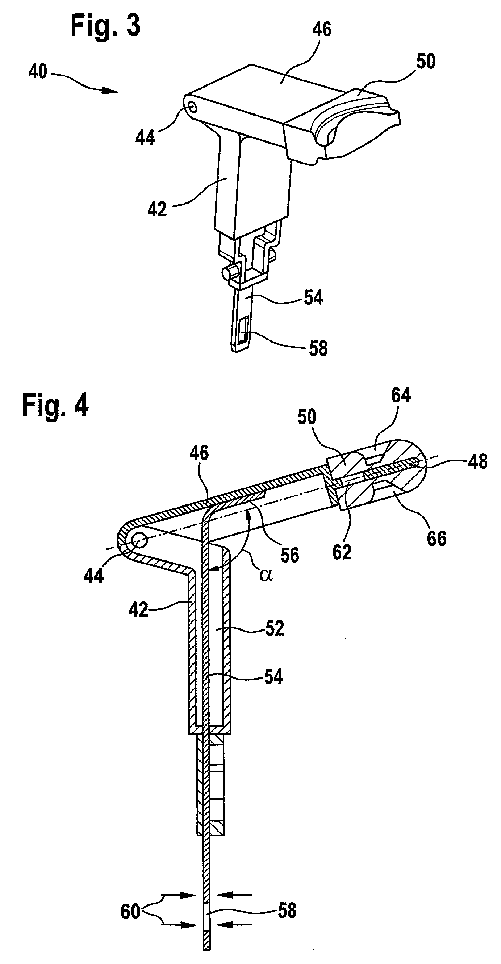

Fig. 3 zeigt eine perspektivische Ansicht einer allgemein mit 40 bezeichneten Aufbissvorrichtung nach einem Ausführungsbeispiel der Erfindung in schematischer Darstellung, Fig. 4 einen Schnitt durch die Aufbissvorrichtung 40 von Fig. 3. Die Aufbissvorrichtung 40 weist einen Halter 42 auf, an den über ein Gelenk 44 eine dünne Platte 46 schwenkbar angelenkt ist. Auf ihrer vom Gelenk 44 abgewandten Seite geht die dünne Platte 46 in einen parallel zur dünnen Platte ausgebildeten Aufnahmebereich 48 über.

Der Aufnahmebereich 48 weist eine Ausnehmung 62 auf, mittels deren in weiter unten genauer beschriebener Weise ein auswechselbarer Aufbisseinsatz 50 schnell und unkompliziert an der dünnen Platte befestigt werden kann. An seiner Ober- und Unterseite ist der Aufbisseinsatz 50 jeweils mit einer Bissrille 64 bzw. 66 versehen, die jeweils den Zahnbogen von Ober- bzw. Unterkiefer des Patienten aufnehmen. Durch diese Gestaltung ist sichergestellt, dass die dünne Platte 46 parallel zur Kauebene des Patienten liegt, wenn er bei der Röntgenaufnahme in den Aufbisseinsatz 50 beißt.

Der Schwenkwinkel α der dünnen Platte 46 wird über eine auf- und abbewegliche Schiene 54 gemessen, die in einem Bereich 56 mit der dünnen Platte 46 verbunden ist, und im Inneren 52 des Halterteils 42 senkrecht nach unten verläuft. In ihrem unteren Abschnitt ist die Schiene 54 mit einem Loch 58 versehen. Die Höhenlage des Loches 58 wird durch zwei Lichtschranken ermittelt, die durch die Pfeile 60 angedeutet sind. Aus der Höhenlage kann auf den Schwenkwinkel der Platte 46 zurückgeschlossen werden. Durch die Übertragung der Neigung der Platte 46 auf die bewegliche Schiene wird die Messung des Schwenkwinkels α weit nach unten in einen strahlungsfreien Bereich des Halters 42 verlegt. Der Röntgenbereich kann damit weitestgehend metallfrei gehalten werden.

Die Aufbissvorrichtung wirkt mit einer in Fig. 1 dargestellten Antriebseinrichtung 15 zusammen, so daß die Schiene 54 auf- oder abbewegt wird und damit die dünne Platte 46 in eine gewünschte Position gebracht werden kann. Durch die mit der Höhenverstellung einhergehende Verschwenkung der dünnen Platte 46 wird der Kopf des Patienten sanft in geführt, bis er die korrekte Neigung für die Panorama-Röntgenaufnahme erreicht hat.

Die Höhenverstellung der Platte 46 kann dabei interaktiv durch den Bediener erfolgen. Beispielsweise kann der Bediener solange auf einen Justierknopf drücken, bis die gewünschte Neigung erreicht ist. Das Erreichen einer vorher eingestellten Winkelstellung, beispielsweise α = 105°, die einem Winkel zwischen Patte 46 und der Horizontalen von 15° entspricht, kann durch optische und/oder akustische Signale angezeigt werden.

Alternativ kann der Antrieb die Platte 46 automatisch in die eingestellte Winkelstellung fahren und bei Erreichen der gewünschten Position anhalten. Zusätzlich kann die Winkelstellung der Platte zur Kontrolle für den Bediener angezeigt werden.

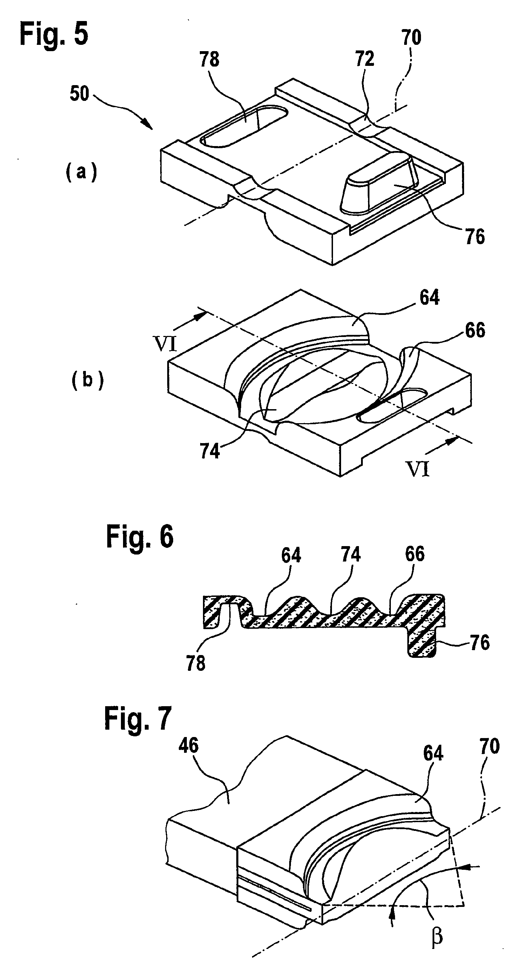

Der in den Figuren 3 und 4 gezeigte Aufbisseinsatz 50 wird nun mit Bezug auf die Figuren 5 bis 7 näher beschrieben. Dabei zeigt Fig. 5 in (a) und (b) eine perspektivische Ansicht des aufgeklappten Einsatzes, gesehen in Fig. 5(a) schräg von unten und in Fig. 5(b) schräg von oben. Figur 6 zeigt einen Schnitt durch den Aufbisseinsatz entlang der Linie VI-VI der Fig. 5(b) und Fig. 7 stellt den Aufbisseinsatz in zusammengeklappter Stellung dar, wie er an der Aufbissvorrichtung der Fig. 3 angebracht ist.

Der Aufbisseinsatz 50 ist im Ausführungsbeispiel einstückig aus geschlossenzelligem Ethylenschaum gefertigt, einem weichen, weitgehend röntgenstrahlendurchlässigem Material. An seiner Oberseite weist der Aufbisseinsatz 50 Bissrillen 64 und 66 auf, die einen Teil des Zahnbogens von Ober- und Unterkiefer des Patienten aufnehmen. Der Aufbisseinsatz 50 kann entlang einer mittigen Faltkante 70 zusammengeklappt werden, welche durch Einkerbungen 72 auf der Unterseite und eine Mittelrille 74 auf der Oberseite des Einsatzes festgelegt ist.

Auf seiner Unterseite weist der Aufbisseinsatz 50 einen sich keilförmig verjüngenden Vorsprung 76 auf, der beim Zusammenklappen in eine auf der gegenüberliegenden Seite des Einsatzes angeordnete entsprechende Vertiefung 78 eingreift und eine stabile, aber leicht lösbare Verbindung herstellt. Der flächige Aufnahmebereich 48 der Aufbissvorrichtung enthält eine Ausnehmung 62 (Fig. 4), durch die der Vorsprung 76 beim Anbringen des Aufbisseinsatzes hindurchgreift, so dass der Einsatz 50 nach dem Zusammenklappen mit der dünnen Platte 46 der Aufbissvorrichtung fest verbunden ist. Nach der Benutzung kann der Einsatz jedoch ohne große Kraftanstrengung wieder aufgeklappt und aus Hygienegründen weggeworfen werden.

Der im Ausführungsbeispiel der Figuren 5 bis 7 gezeigte Einsatz nimmt bei einer Breite von 40 mm einen Winkelbereich β des Zahnbogens von etwa 30° auf. Um der unterschiedlichen Kiefergröße der Patienten gerecht zu werden, werden Aufbisseinsätze auch in anderen Breiten hergestellt und verwendet. Die unterschiedlichen Breiten lassen sich dabei durch verschiedene Farbmarkierungen oder andere Kennzeichnungen für den Verwender leicht unterscheiden. Mit einem derartigen Aufbisseinsatz erfolgt die Fixierung des Patienten sehr exakt und dennoch für den Patienten komfortabel.

### Bezugszeichenliste

- 10: Röntgenaufnahmeeinrichtung
- 12: Einheit
- 14: Strahlenquelle
- 15: Antriebseinrichtun
- 16: Blende
- 18: Detektorkamera
- 20: Blende
- 22: Röntgenstrahlen
- 24: Kopf
- 26: Auswerteeinheit
- 30: Tomographiezone
- 32: Kieferbogenkamm
- 40: Aufbissvorrichtung
- 42: Halter
- 44: Gelenk
- 46: Platte
- 48: Aufnahmebereich
- 50: Aufbisseinsatz
- 52: Inneres des Halterteils 42
- 54: Schiene
- 56: Bereich
- 58: Loch
- 60: Pfeile
- 62: Ausnehmung
- 64: Bissrille
- 66: Bissrille
- 70: Faltkante
- 72: Einkerbungen
- 74: Mittelrille
- 76: Vorsprung
- 78: Vertiefung

## Patentansprüche

1. Aufbissvorrichtung zur korrekten Positionierung eines Patienten bei einer Aufnahme mit einem Panorama-Röntgengerät mit einem zum Röntgengerät richtungsfest anordenbarem Halterteil (42),wobei das Halterteil (42) eine über ein Gelenk (44) gegen das Halterteil schwenkbare Platte (46) aufweist, die ein Aufbissteil (50) aufweist, in das der Patient beißen kann, und mit Mitteln (58, 60) zum Erfassen des Schwenkwinkels α zwischen der Platte (46) und dem Halterteil (42), die in einem bei einer Röntgenaufnahme strahlenfreien Bereich der Aufbissvorrichtung (40) angeordnet sind, **dadurch gekennzeichnet, dass** die Aufbissvorrichtung Antriebsmittel (15) zur Höhenverstellung des Halterteils (42) und damit zum Verschwenken der den Patienten durch Beißen in das Aufbissteil fixierenden Platte um das Gelenk (44) aufweist, und dass die Antriebsmittel (15) ausgebildet sind, die Platte (46) automatisch in eine vorbestimmte Winkelposition zu fahren und bei Erreichen der vorbestimmten Winkelposition anzuhalten.

2. Aufbissvorrichtung nach Anspruch 1, **dadurch gekennzeichnet, dass** die Mittel (58, 60) zum Erfassen des Schwenkwinkels α einen oder mehrere im Halterteil (42) angeordnete Sensoren umfassen.

3. Aufbissvorrichtung nach Anspruch 1 oder 2, **dadurch gekennzeichnet, dass** die Aufbissvorrichtung Mittel zur Anzeige der Winkelstellung der verschwenkbaren Platte (46) aufweist.

4. Aufbissvorrichtung nach einem der Ansprüche 1 bis 3, **dadurch gekennzeichnet, dass** die Antriebsmittel das Erreichen der vorbestimmten Winkelposition der Platte (46) durch optische und/oder akustische Signale anzeigen.

5. Aufbissvorrichtung nach einem der Ansprüche 1 bis 4, **dadurch gekennzeichnet, dass** die schwenkbare Platte (46) mit einer im Halterteil (42) auf- und abbeweglichen Schiene (54) verbunden ist, die eine Ausnehmung (58) zur Positionsbestimmung der Schiene (54) im Halterteil (42) aufweist.

6. Aufbissvorrichtung nach einem der Ansprüche 1 bis 5, **dadurch gekennzeichnet, dass** das Aufbissteil mit einer auswechselbaren Schutzhülle versehen ist.

7. Aufbissvorrichtung nach einem der Ansprüche 1 bis 5, **dadurch gekennzeichnet, dass** das Aufbissteil durch einen auswechselbaren Aufbisseinsatz (50) gebildet ist.

8. Aufbissvorrichtung nach Anspruch 7, **dadurch gekennzeichnet, dass** der Aufbisseinsatz (50) aus einem weichen Material gebildet ist.

9. Aufbissvorrichtung nach Anspruch 7 oder 8, **dadurch gekennzeichnet, dass** der Aufbisseinsatz (50) aus einem im Wesentlichen röntgenstrahlendurchlässigen Material gebildet ist.

10. Aufbissvorrichtung nach einem der Ansprüche 7 bis 9, **dadurch gekennzeichnet, dass** der Aufbisseinsatz (50) aus geschlossenzelligem Ethylenschaum gefertigt ist.

11. Aufbissvorrichtung nach einem der Ansprüche 7 bis 10, **dadurch gekennzeichnet, dass** der Aufbisseinsatz (50) einen Winkelbereich β des Zahnbogens aufnimmt, der zwischen 20° und 40°, insbesondere bei etwa 30° liegt.

12. Aufbissvorrichtung nach einem der Ansprüche 7 bis 11, **dadurch gekennzeichnet, dass** der Aufbisseinsatz (50) auf seiner Oberseite und seiner Unterseite jeweils eine Bissrille (64, 66) zur Aufnahme des Zahnbogens von Ober- und Unterkiefer des Patienten aufweist.

13. Aufbissvorrichtung nach einem der Ansprüche 7 bis 12, **dadurch gekennzeichnet, dass** der Aufbisseinsatz (50) einstückig und entlang einer Faltkante (70) klappbar ausgebildet ist.

14. Aufbissvorrichtung nach einem der Ansprüche 7 bis 13, **dadurch gekennzeichnet, dass** der Aufbisseinsatz (50) an gegenüberliegenden Seiten einen sich keilförmig verjüngenden Vorsprung (76) und eine entsprechende Vertiefung (78) zur Aufnahme des Vorsprungs (76) aufweist, zum lösbaren Aufstecken des Aufbisseinsatzes (50) auf die schwenkbare Platte (46).

15. Verfahren zur korrekten Positionierung eines Patienten bei einer Aufnahme mit einem Panorama-Röntgengerät unter Verwendung einer Aufbissvorrichtung mit einem zum Röntgengerät richtungsfest anordenbarem Halterteil (42), wobei das Halterteil (42) eine über ein Gelenk (44) gegen das Halterteil schwenkbare Platte (46) aufweist, die ein Aufbissteil (50) aufweist, in das der Patient beißt, mit Mitteln (58, 60) zum Erfassen des Schwenkwinkels α zwischen der Platte (46) und dem Halterteil (42), die in einem bei einer Röntgenaufnahme strahlenfreien Bereich der Aufbissvorrichtung (40) angeordnet sind, und mit Antriebsmitteln (15) zur Höhenverstellung des Halterteils (42) und damit zum Verschwenken der den Patienten durch Beißen in das Aufbissteil (50) fixierenden Platte (46) um das Gelenk (44), wobei die Antriebsmittel (15) die Platte (46) automatisch in eine vorbestimmte Winkelposition fahren und bei Erreichen der vorbestimmten Winkelposition anhalten.

16. Verfahren nach Anspruch 15, **dadurch gekennzeichnet, dass** die Antriebsmittel das Erreichen der vorbestimmten Winkelposition der Platte (46) durch optische und/oder akustische Signale anzeigen.

## Claims

1. A bite block assembly for correct positioning of a patient while taking a radiograph with a panoramic X-ray device, comprising a holding member (42) that can be located in a directionally stable position relative to said X-ray device, which holding member comprises a plate (46) that can be pivoted relatively to said holding member about a pivot (44) and exhibits a bite piece (50), on which the patient can bite, and comprising means (58, 60) for detecting the angle of deflection α between said plate (46) and said holding member (42), which means are disposed in a region of the bite block assembly (40) that is free from X-ray irradiation when the radiograph is being taken, **characterized in that** said bite block assembly comprises a driving system for vertically adjusting said holding member (42) and thus for pivoting said plate about said pivot (44) for the purpose of fixing the position of the patient biting on said bite piece, and that said driving system (15) is adapted to move said plate (46) automatically into a predefined angular position and to stop when said predefined angular position is attained.

2. A bite block assembly as defined in claim 1, **characterized in that** said means (58, 60) for detecting the angle of deflection α comprise one or more sensors disposed in said holding member (42).

3. A bite block assembly as defined in claim 1 or claim 2, **characterized in that** said bite block assembly comprises means for indicating the angular position of the pivotal plate (46).

4. A bite block assembly as defined in claim 1 or claim 2, **characterized in that** said driving system (15) indicates when said predefined angular position of said plate (46) has been attained by means of optical and/or acoustic signals.

5. A bite block assembly as defined in any one of the previous claims, **characterized in that** said pivotal plate (46) is connected to a rail (54) which is disposed in said holding member (42), is capable of being moved upwardly and downwardly and has an opening (58) for the purpose of positioning said rail (54) in said holding member (42).

6. A bite block assembly as defined in any one of the previous claims, **characterized in that** said bite piece is provided with a replaceable protective sheath.

7. A bite block assembly as defined in any one of claims 1 to 5, **characterized in that** said bite piece is in the form of a replaceable occlusal insert (50).

8. A bite block assembly as defined in claim 7, **characterized in that** said occlusal insert (50) is of a soft material.

9. A bite block assembly as defined in claim 7 or claim 8, **characterized in that** said occlusal insert (50) is of a substantially radiolucent material.

10. A bite block assembly as defined in any one of claims 7 to 9, **characterized in that** said occlusal insert (50) is made of closed-cell ethylene foam.

11. A bite block assembly as defined in any one of claims 7 to 10, **characterized in that** said occlusal insert (50) occupies an angle β of the dental arch which ranges from 20° to 40° and is, in particular, approximately 30°.

12. A bite block assembly as defined in any one of claims 7 to 11, **characterized in that** said occlusal insert (50) has, on its upper surface and on its undersurface respectively, a bite groove (64, 66) for the accommodation of the dental arch of the upper and lower jaws of the patient.

13. A bite block assembly as defined in any one of claims 7 to 12, **characterized in that** said occlusal insert (50) is a unitary piece and is foldable along a folding edge (70).

14. A bite block assembly as defined in any one of claims 7 to 13, **characterized in that** said occlusal insert (50) has, on opposite sides thereof, a wedge-shaped tapered projection (76) and a complementary recess (78) for the accommodation of said projection (76) for releasably fitting said occlusal insert (50) to said pivotal plate (46).

15. A method for correctly positioning a patient while taking a radiograph with a panoramic X-ray device, using a bite block assembly comprising a holding member (42) that can be located in a directionally stable position relative to said X-ray device, which holding member comprises a plate (46) that can be pivoted relatively to said holding member about a pivot (44) and exhibits a bite piece (50), on which the patient bites, and comprising means (58, 60) for detecting the angle of deflection α between said plate (46) and said holding member (42), which means are disposed in a region of the bite block assembly (40) that is free from X-ray irradiation when the radiograph is being taken, and comprising a driving system for vertically adjusting said holding member (42) and thus for pivoting said plate about said pivot (44) for the purpose of fixing the position of the patient biting on said bite piece, said driving system (15) being adapted to move said plate (46) automatically to a predefined angular position and to stop when said predefined angular position is attained.

16. A method as defined in claim 15, **characterized in that** said driving system (15) indicates when said predefined angular position of said plate (46) has been attained by means of optical and/or acoustic signals.

## Revendications

1. Dispositif à mordre garantissant un positionnement correct du patient lors d'une radiographie panoramique, comprenant un élément support (42) pouvant être orienté de manière fixe par rapport à l'appareil de radiographie, sachant que l'élément support (42) présente une plaque (46) pouvant pivoter par rapport à l'élément support par le biais d'une articulation (46), présentant une pièce à mordre (50) que le patient doit serrer avec ses dents, ainsi que des moyens (58, 60) permettant de déterminer l'angle de pivotement α entre la plaque (46) et l'élément support (42), qui sont disposés dans une zone sans rayonnements du dispositif à mordre (40) lors d'une radiographie, sachant que le dispositif à mordre présente des moyens d'entraînement (15) pour régler en hauteur l'élément support (42) et ainsi pour faire pivoter la plaque fixant le patient mordant dans la pièce à mordre sur l'articulation (44) et en ce que les moyens d'entraînement (15) sont formés pour diriger la plaque (46) automatiquement dans une position angulaire prédéterminée et pour l'arrêter lorsque la position angulaire prédéterminée est atteinte.

2. Dispositif à mordre selon la revendication 1, **caractérisé en ce que** les moyens (58, 60) comprennent un ou plusieurs détecteur(s) disposé(s) dans l'élément support (42) pour détecter l'angle de pivotement α.

3. Dispositif à mordre selon la revendication 1 ou 2, **caractérisé en ce qu'**il présente des moyens d'affichage de la position angulaire de la plaque (46) pouvant pivoter.

4. Dispositif à mordre selon l'une des revendications 1 à 3, **caractérisé en ce que** les moyens d'entraînement affichent la position angulaire prédéterminée atteinte par la plaque (46) par le biais de signaux optiques et/ou acoustiques.

5. Dispositif à mordre selon l'une des revendications 1 à 4, **caractérisé en ce que** la plaque (46) pouvant pivoter est reliée à un rail (54) mobile vers le haut et vers le bas dans l'élément support (42), lequel présente un évidement (58) pour déterminer la position du rail (54) dans l'élément support (42).

6. Dispositif à mordre selon l'une des revendications 1 à 5, **caractérisé en ce que** la pièce à mordre est munie d'une enveloppe de protection amovible.

7. Dispositif à mordre selon l'une des revendications 1 à 5, **caractérisé en ce que** la pièce à mordre est formée par un insert à mordre (50) amovible.

8. Dispositif à mordre selon la revendication 7, **caractérisé en ce que** l'insert à mordre (50) est fabriqué dans un matériau souple.

9. Dispositif à mordre selon la revendication 7 ou 8, **caractérisé en ce que** l'insert à mordre (50) est fabriqué dans un matériau essentiellement transparent aux rayons X.

10. Dispositif à mordre selon l'une des revendications 7 à 9, **caractérisé en ce que** l'insert à mordre (50) est fabriqué dans une mousse d'éthylène à cellules fermées.

11. Dispositif à mordre selon l'une des revendications 7 à 10, **caractérisé en ce que** l'insert à mordre (50) adopte une plage angulaire β de l'arcade dentaire, qui est d'environ 20° à 40°, de préférence d'environ 30°.

12. Dispositif à mordre selon l'une des revendications 7 à 11, **caractérisé en ce que** l'insert à mordre (50) présente sur sa surface supérieure et sa surface inférieure respectivement une nervure à mordre (64, 66) pour recevoir l'arcade dentaire du maxillaire et de la mandibule du patient.

13. Dispositif à mordre selon l'une des revendications 7 à 12, **caractérisé en ce que** l'insert à mordre (50) est monobloc et rabattable le long d'une arête de pliage (70).

14. Dispositif à mordre selon l'une des revendications 7 à 13, **caractérisé en ce que** l'insert à mordre (50) présente sur des côtés opposés une saillie (76) se rétrécissant de manière tronconique et un renfoncement (78) correspondant pour recevoir la saillie (76), pour insérer de manière séparable l'insert à mordre (50) sur la plaque (46) pouvant pivoter.

15. Procédé pour le positionnement correct d'un patient lors d'une radiographie panoramique, utilisant un dispositif à mordre comprenant un élément support (42) pouvant être orienté de manière fixe par rapport à l'appareil de radiographie, sachant que l'élément support (42) présente une plaque (46) pouvant pivoter par rapport à l'élément support par le biais d'une articulation (46), présentant une pièce à mordre (50) que le patient doit serrer avec ses dents, ainsi que des moyens (58, 60) permettant de déterminer l'angle de pivotement α entre la plaque (46) et l'élément support (42) disposés dans une zone sans rayonnements du dispositif à mordre (40) lors d'une radiographie, et comprenant des moyens d'entraînement (15) pour régler en hauteur l'élément support (42) et ainsi pour faire pivoter la plaque fixant le patient mordant dans la pièce à mordre sur l'articulation (44) sachant que les moyens d'entraînement (15) sont formés pour diriger la plaque (46) automatiquement dans une position angulaire prédéterminée et pour l'arrêter lorsque la position angulaire prédéterminée est atteinte.

16. Procédé selon la revendication 15, **caractérisé en ce que** les moyens d'entraînement affichent la position angulaire prédéterminée atteinte par la plaque (46) par le biais de signaux optiques et/ou acoustiques.
